# EUROPEAN PATENT SPECIFICATION

(11) **EP 4 091 660 B1**
(45) Date of publication and mention of the grant of the patent: **04.10.2023**
(21) Application number: 21425026.8
(22) Date of filing: 20.05.2021
(51) Int. Cl.: A61N 1/04, A61N 1/02, A61N 1/36

(54) **ELECTROSTIMULATOR FOR VISION**
ELEKTROSTIMULATOR FÜR DIE SEHKRAFT
ÉLECTROSTIMULATEUR POUR LA VISION

(43) Date of publication of application: 23.11.2022
(73) Proprietor: Subvision S.r.l., 20149 Milano (IT)
(72) Inventor: Modrian, Mauro, 25038 Rovato (BS) (IT); Ferrari, Giorgio, 25132 Brescia (IT)
(74) Representative: Riccardi, Elisa

(56) References cited:
- US-A- 4 614 193
- US-A- 5 522 864
- US-A1- 2017 266 446
- US-A1- 2019 008 410
- US-A1- 2020 171 307

## Description

The present invention relates to an electrostimulator for vision.

Unfortunately, several sight disorders are known, either innate or not. Just to cite some, Age-related Macular Degeneration- AMD, presbyopia, diabetic retinopathy, retinitis pigmentosa and glaucoma are mentioned.

It is believed that a suitable electric stimulation of the periocular area has beneficial effects on some sight disorders.

US7,251,528B2 discloses a non-invasive therapy for vision disorders, comprising: positioning a first electrode on or proximate to an eyelid of a subject; positioning a second electrode on the subject; generating an electric current between said electrodes of between 1-800 microamps (assuming a resistance of about 500 ohm), wherein the electrical current is generated at multiple frequencies and modulated with a carrier signal in a range of about 10,000 hertz to less than 12,000 hertz. The electrical current is generated, for example, at about 292 hertz for about one minute; at about 8-9 hertz for at least seven minutes; at about 0.15-0.3 hertz for at least seven minutes; and/or at about 31 Hz for about two minutes. The carrier signal is modulated on and off and is inverted by reversing a polarity thereof at said electrodes, in particular about every 0.05 or 0.5 seconds.

The document further discloses a direct current waveform generator, that produces an amplitude modulated, low level, pulsed, direct current applied between electrodes, one placed on or proximally to a closed eyelid and the other one on a remote body location, for example a top of the subject's corresponding hand.

Further electrostimulators for visions are disclosed for example in US 4,614,193**,** in US 6,035,236 B1 and US6,275,735 B1**,** which use various types of circuits for generating the treatment currents. US4,989,605 also discloses an electrostimulator for mammalian treatment.

US 5,522,864 discloses a DC waveform generator for generating a direct current for treating i.a. sight disorders, which uses a first opto-isolator which phototransistor is interposed between the battery power source and a voltage regulator, and possibly a second opto-isolator for activating a timer so that only the time when the electrodes are actually connected is computed.

US-A-2020/171307 discloses a patient treatment unit according to the preamble of claim 1.

The Applicant faced the technical problem of providing an electrostimulator for vision which is capable of generating treatment voltage waveforms in a particularly precise and repeatable manner. This objective should be obtained also at a very low modulating frequency. Another object is that of ensuring the patient's safety and safeguarding the components of the driver against the circulation of currents along undesired paths.

The subject-matter disclosed herein relates to an electrostimulator for vision according to claim 1.

The use of a microcontroller within the waveform generator allows the waveforms applied during the treatment to be generated in a very accurate manner and with a precise repeatability. Through the provision of at least one opto-isolator or photocoupler for electrically insulating the electrodes from a power supply of the electrostimulator, signal coupling is performed with a pass band that, starting from direct, reaches up to a few tens of KHz. Distortions of the generated waveforms are thus advantageously avoided, which might occur, especially at low modulating frequencies, if the coupling were of an inductive type. Furthermore, the patient's safety against the circulation of currents along undesired paths is ensured.

Because the waveform generator comprises a microcontroller and at least one electrode driver which comprises said at least one photo-coupler at the interface with the microcontroller, the electrically isolated coupling occurs between the control signals emitted by the microcontroller and all of the other components of the driver.

Further features and advantages of the subject-matter disclosed herein will better appear from the following detailed description of some preferred embodiments thereof, made with reference to the attached drawings, wherein:
- FIG. 1 is an isometric view of an electrostimulator for vision illustrative of the subject-matter disclosed herein,
- FIG. 2 shows a block diagram of an electrostimulator for vision illustrative of the subject-matter disclosed herein,
- FIG. 3 shows a block diagram of a power supply of an electrostimulator for vision 1 illustrative of the subject-matter disclosed herein,
- FIG. 4 shows a block diagram of an electrode driver of an electrostimulator for vision illustrative of the subject-matter disclosed herein,
- FIG. 5 shows some waveforms of signals of an electrostimulator in conformity with FIGs. 2-4 or useful for describing the operation thereof, with a first set of values of respective parameters, and
- FIG. 6 shows some waveforms of signals of an electrostimulator in conformity with FIGs. 2-4 or useful for describing the operation thereof, with a second set of values of respective parameters.

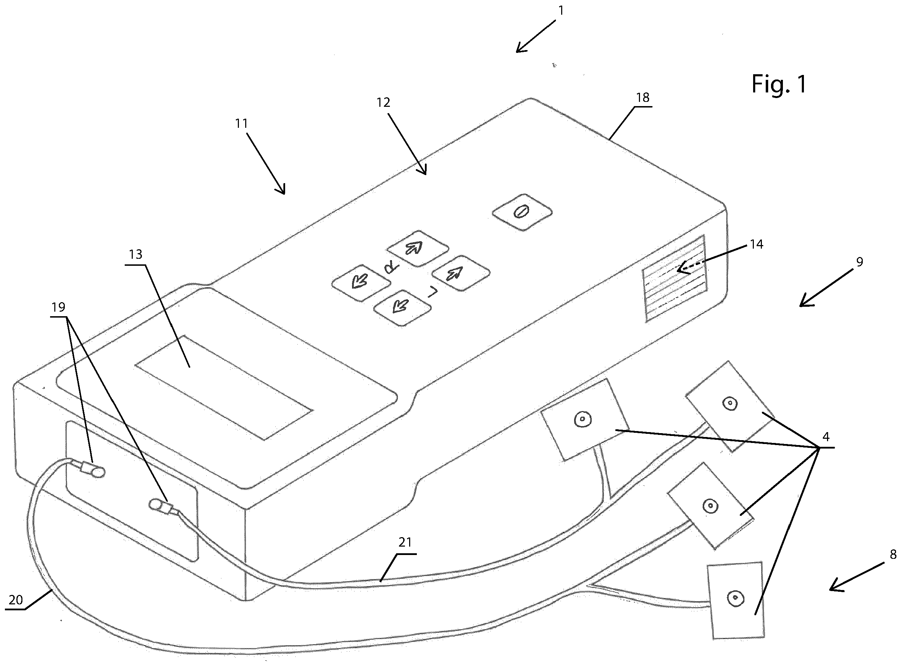

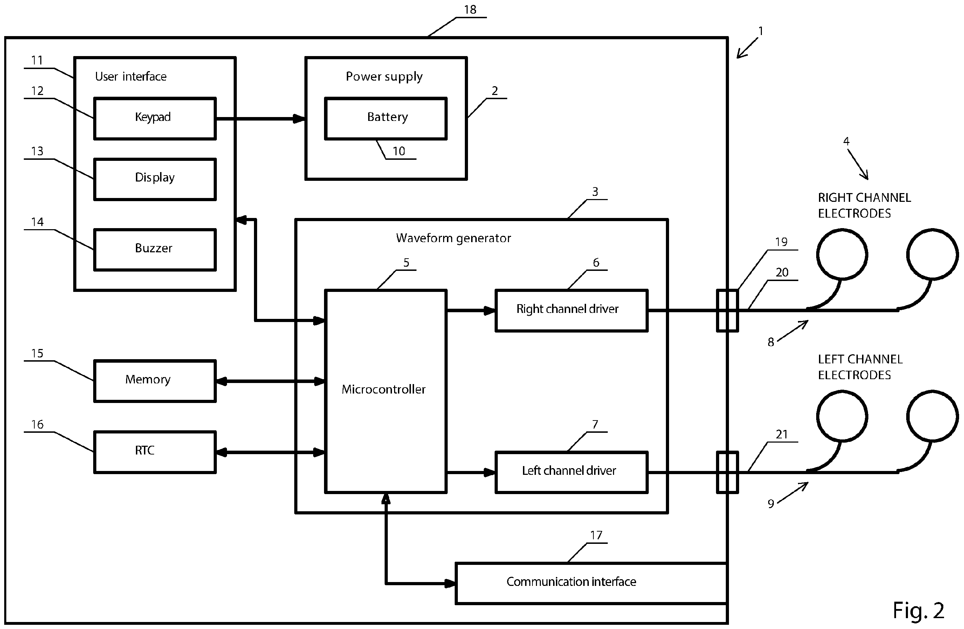

With reference first to **FIGs. 1** **and** **2****,** an electrostimulator for vision 1 comprises a voltage power supply 2, a waveform generator 3 and at least one pair of electrodes 4 for applying a treatment voltage to a body zone to be treated, and thus for generating a treatment current in the body zone. The electrodes 4 are configured to make contact with the skin of a subject to be treated, and are preferably of a nonallergenic and self-adhesive-to-skin type, and optionally covered by a one-use film.

In the use of the electrostimulator 1 for treating sight disorders, one of the two electrodes 4 of the single or each pair 8, 9 is applied to the periocular zone, or, alternatively, onto a closed eyelid, of an eye of the subject to be treated, and the other electrode 4 of the same pair 8, 9 is applied to the skin of the subject to be treated in a location remote from the eyelid, preferably on the back of the ipsilateral hand. A voltage in conformity with one or more desired waveform(s), generated by the waveform generator 3, is applied to the electrodes 4, and a treatment current flows through the electrodes, the human body and the electrostimulator. In the present description and in the attached claims, term "voltage" is sometimes also used to indicate a voltage difference.

As better explained hereinbelow, the treatment voltage - and thus the current - is preferably of a pulsed, discontinuous, and bipolar type. In general, the current intensity depends on the resistance offered by the subject's body between the electrodes 4 to which the treatment voltage is applied. Through a suitable sizing of the electric components of the waveform generator 3, it is possible, if desired, to cause the treatment current to have an intensity which is independent or almost independent of the resistance offered by the human body, at least within some limits of change of such a resistance.

The waveform generator 3, better described hereinbelow, preferably comprises a microcontroller 5 and at least one driver 6, 7 of the electrodes 4.

In the present description and in the attached claims, under term "microcontroller" a microprocessor comprising within it also a certain amount of memory and various integrated peripheral devices, such as timers, analog-to-digital converters, etc., is meant to be indicated.

Preferably, the waveform generator 3 is configured to emit on two channels: a right channel and a left channel. The waveform emitted on a channel at a given time may be different, in one or more of its parameters or configurable variables, from the waveform emitted on the other channel. In this case there are two pairs 8, 9 of electrodes 4, one provided for the left channel, and the other one provided for the right channel. For each pair 8, 9 of electrodes there is, in the above-mentioned embodiment, a respective driver 6, 7 of the electrodes, so as to be able to perform a simultaneous treatment of both eyes of a patient. The driver 6, 7 and the electrodes 4 of pairs 8, 9 are named right and left, it being understood that if there is one single channel, it is intended for non-simultaneous use on the right side and on the left side. Hereinbelow, for the sake of simplicity, reference will be mainly made to only one pair 8 of electrodes 4 and to the respective driver 6.

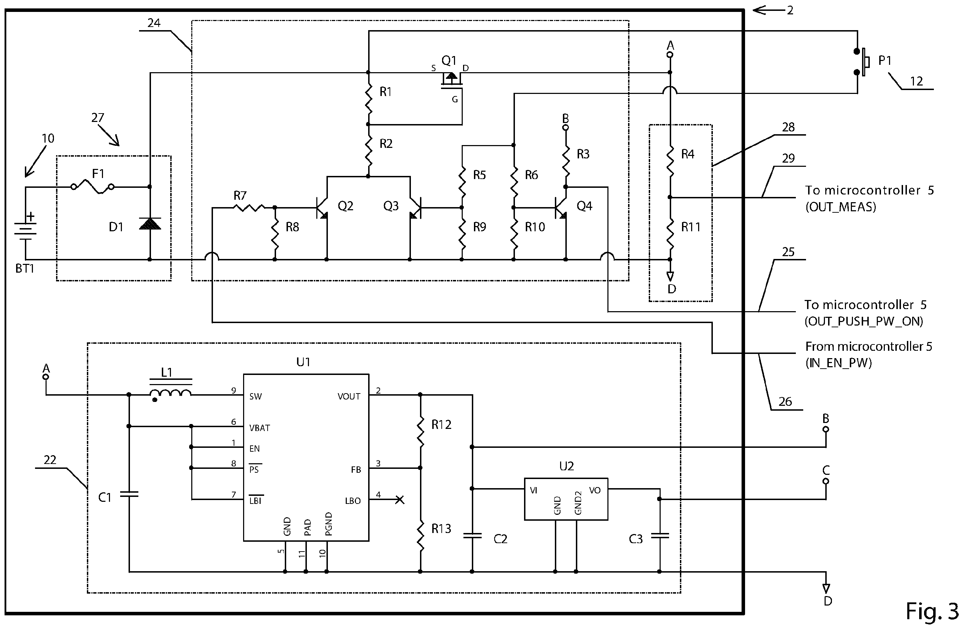

The voltage supply 2 is preferably configured to provide one and preferably two stable voltages, at a different voltage, for supplying the other components of the electrostimulator 1. The electric connections for supplying the various components by the power supply 2 are not shown for the sake of simplicity. An embodiment of the power supply is described later with reference to FIG. 3.

The voltage power supply 2 preferably comprises a power source 10, more preferably of the battery type of possibly rechargeable cells, so as to make the electrostimulator 1 completely portable. Hereinbelow, reference will be mainly made, merely by way of a non-limiting example, to such a voltage power supply 2 and the power source 10 will be sometimes indicated merely as battery 10. However, also supply from the mains might be provided for.

The electrostimulator 1 preferably further comprises a user interface 11.

The user interface 11 comprises input means, preferably of the keypad 12 type, and output means, preferably of the display 13 type, and possibly further comprising a buzzer 14 or beeper.

The keypad 12 preferably comprises a key for switching the electrostimulator on and off, and one or more keys for setting the value of one or more variables, among which in particular the amplitude of the voltage at the electrodes 4 and therefore the amplitude of the treatment current, preferably separately for the right channel and the left channel. The on/off key may be absent in the case of supply from the mains, or when the cells of battery 10 are easily removable, the switching on and off being controlled by insertion and removal of the plug or of the cells.

The keys of keypad 12 are operatively connected to respective inputs of the microcontroller 5 of the waveform generator 3. The on/off key is also operatively connected to the power supply 10, as better described hereinbelow.

Display 13, if provided for, allows data and useful indications relating to the treatment signal(s) being delivered, to the setting of the electrostimulator 1, etc., to be displayed. The keypad 12 and the display 13 may also be combined into a touch screen.

Preferably the electrostimulator 1 further comprises memory means 15 and/or a real time clock, RTC, 16 and/or a communication interface 17.

The memory means 15 are configured to store data collected during the treatments during use of the electrostimulator 1. The memory means 15 may also be configured to store instructions and values of constants and variables useful for the operation of the microcontroller 5 or of other components of the electrostimulator 1, which are not stored in a memory internal to the microcontroller 5 itself.

The RTC 16 is preferably powered, besides by the power supply 2, also by a dedicated backup battery (not shown), so that during the replacement of the cells of the battery 10, the data and time setting is not lost.

The memory means 15 and the RTC 16 allow a journal or *"data logger"* functionality to be embodied.

Furthermore, the RTC 16 may also be used to cause the apparatus to switch on at predetermined times, for example causing the buzzer 14 to play and/or displaying on the display 13 a memorandum to remind the patient when to make the treatments.

The communication interface 17, for example with a tablet, a smartphone, a computer or a server, may be whatever one, and preferably is of the wireless type, more preferably at short range, for example according to Bluetooth protocol.

The communication interface 17 is provided to communicate data collected by the microcontroller 5 and/or stored in the memory means 15, possibly together with the respective data and time indication of the RTC 16 in the above-mentioned journal. The communication interface 17 may be further provided for receiving commands and/or setting parameters of the microcontroller 5 from outside of the electrostimulator 1.

All the interaction with the electrostimulator 1 might also take place remotely, through a tablet, a smartphone, a computer or a server, in that case the user interface 11 could even be totally absent.

The above-mentioned components are housed in a casing 18, which is preferably of the portable type. The electrodes 4 of each pair 8, 9 are preferably connected in a removable manner to the casing 18, for example by means of plug connectors 19 or standard two-contact jacks, at the end of a respective conductor cable 20, 21 opposite that to which the electrodes are connected 4.

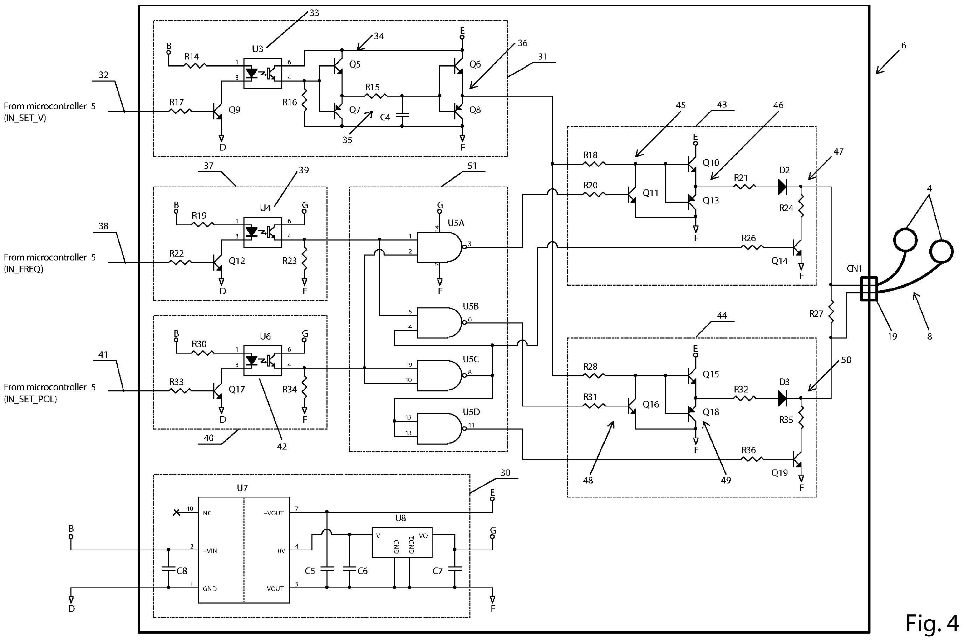

As better described hereinbelow with reference to FIG. 4, waveform generator 3 comprises at least one photocoupler for electrically isolating said at least one pair of electrodes 4 from said voltage power supply 2. Thus, each pair 8, 9 of electrodes 4 is electrically isolated with respect to the power source, for example with respect to the battery 10, so that the treatment current flows in a closed circuit comprising the electrically isolated portion of the waveform generator 3, the electrodes 4 and the human body portion between the application sites of the electrodes 4. When both pairs 8, 9 of electrodes 4 are provided for, one or more electrical isolation photocouplers are advantageously arranged in such a manner that the two pairs 8, 9 of electrodes 4 are also electrically isolated from each other.

In this manner, the body region involved by the treatment current(s) is controlled by the location of the electrodes 4 and leakage currents from the electrostimulator 1 to the patient are avoided, in case for example of a not perfectly isolated casing 18, and/or from an electrode of one pair to an electrode of the other pair, which could involve body regions where an electric current is undesired.

As mentioned, the waveform generator 3 comprises, preferably, a microcontroller 5 and at least one driver 6, 7 of electrodes 4. In this case, the at least one electric isolation photocoupler is preferably provided for in each driver 6, 7 of the electrodes 4, at the interface with the microcontroller 5, as better described below with reference to FIG. 4.

The microcontroller 5 manages the operation of the electrostimulator 1 as a whole, and in particular controls the driver(s) 6, 7. The use of a microcontroller 5 allows the waveforms applied during the treatment to be generated in a very accurate manner and with a precise repeatability. Those skilled in the art will understand that, alternatively, the functions of the microcontroller 5 described herein could be embodied by electronic components.

Each driver 6, 7 generates, under control of the microcontroller 5, a suitable voltage signal at the electrodes 4 of the respective pair 8, 9, so that between them a treatment current having the desired characteristics flows.

Before describing in detail the waveform generator 3 and in particular the driver 6, the power supply 2 is described, with the aid of **FIG. 3****,** which represents the circuit layout of an example embodiment thereof.

The voltage power supply 2 shown comprises, as mentioned, the battery 10 and is preferably configured to provide one and preferably two stable voltages, at a different voltage, for supplying the other components of the electrostimulator 1. Preferably, the first stable direct voltage is used to power the components of the waveform generator 3, while the second stable direct tension is used to power some components of the electrostimulator 1, for example for powering the buzzer 14 and/or the Bluetooth communication interface 17. In the following, reference will be sometimes made, merely by way of a non-limiting example, to a battery 10 comprising two cells of 1.5 V each (for example two alkaline type "AA" cells) and configured to generate two stable voltages, at +5V and +3.3V, respectively.

In detail, in the embodiment shown, the power supply 2 comprises a voltage stabilizer 22 which generates one and preferably two different stable direct voltages, between nodes B and D and possibly between nodes C and D, starting from the voltage of the battery 10, which is applied, essentially unchanged, between nodes A and D in the manner disclosed shortly below. The node labelled with letter D is at the voltage of the negative terminal of battery 10.

In the case of the example voltage values indicated above, the voltage stabilizer 22 comprises a voltage booster, but in other cases either or both stable voltages may be lower than the voltage delivered by the battery 10.

The voltage stabilizer 22 comprises, in the embodiment shown, a DC/DC step-up converter or boost converter U1, which, starting from a voltage of the battery 10 at node A, for example of +3V, generates a high stable voltage, for example of +5V, at node B. The boost converter U1 is preferably selected so as to be capable of generating the high stable voltage (+5V) even when the cells of battery 10 are very discharged and therefore of allowing all the energy present in the cells of battery 10 to be exploited.

The DC/DC converter U1 is coupled to node A at the positive voltage of battery 10 through inductor L1, in a manner well known *per se* to those skilled in the art.

The voltage stabilizer 22 may also comprise a stabilizer regulator or linear Low DropOut (LDO) converter U2, which, starting from the output voltage of the boost converter U1 (e.g. +5V) generates a second stable direct voltage (e.g. +3.3V), at node C.

In other embodiments, the voltage stabilizer 22 could comprise two voltage regulators operating in parallel rather than cascaded.

The power supply 2 preferably comprises a switching on/off stage 24 operatively connected between the battery 10 -preferably not directly, as better explained below-, the on/off key of keyboard 12, herein labelled as key P1, and input node A of the voltage booster 22.

The switching on/off stage 24 comprises a P-channel MOSFET Q1 which normally is not actuated and behaves as a normally open switch, therefore all the electronics connected downstream within power supply 2, connected to node A, normally is not powered.

In order to switch the electrostimulator 1 on it is necessary to press the on/off key P1: by doing that, the npn transistors Q4 and Q3 are activated. Transistor Q3 controls the gate of pMOS Q1, with consequent activation of pMOS Q1, which therefore behaves as a temporarily closed switch. The positive voltage of the battery 10 is therefore present at node A, and all the electronics of the electrostimulator 1, microcontroller 5 included, is powered, through the voltage booster 22. The collector of npn transistor Q4 is connected to an input of microcontroller 5 through a connection 25. When the microcontroller 5 detects, on that input thereof, the activation of a signal, OUT_PUSH_PW_ON in FIG. 3, indicative of the pressure of the on/off key P1, the microcontroller 5 commands the activation of a signal, IN_EN_PW in FIG. 3, on an output thereof which is connected, through a connection 26, to the switching on/off stage 24. The connection 26 leads, through a resistor R7, to the base of a npn transistor Q2. When the npn transistor Q2 is activated by the signal IN_EN_PW, in turn it activates the pMOS Q1 - which pMOS Q1 is however already activated because the on/off key P1 is still pressed. At this time it is however possible to release the key P1, because the transistor Q2, controlled by the signal IN_EN_PW of the microcontroller 5, inhibits the switching off of the electrostimulator 1.

In order to switch the electrostimulator 1 off, it is necessary to press again the on/off key P1 of keyboard 12: the microcontroller 5, when it detects the consequent activation of the signal OUT_PUSH_PW_ON on the connection 25, deactivates the output IN_EN_PW on connection 26 connected to transistor Q2, thus allowing the transistor Q2 and thus the pMOS Q1 and the electrostimulator 1 as a whole to be switched off, because the voltage of the battery 10 is not present at node A anymore, and therefore the stable voltage(s) is(are) not provided anymore by the voltage booster 22 at node(s) B, C.

The power supply 2 preferably further comprises a protection stage 27 interposed between the battery 10 and the switching on/off stage 24. The protection stage 27 comprises, in the embodiment shown, a resettable protection fuse F1 connected between the positive terminal of the battery 10 and the source of pMOS Q1, as well as a diode D1 having the anode connected to the negative terminal of the battery 10 and the cathode connected to pMOS Q1 and to the positive terminal of the battery 10 through the resettable protection fuse F1. In case of an incorrect insertion of the cells of the battery 10, and therefore of polarity inversion of the battery 10, the diode D1 enters conduction state causing the protection fuse F1 to intervene, thus avoiding damages to the electrostimulator 1. It will be understood that, since the fuse is resettable, in order to restore the normal operation of the electrostimulator 1 it is sufficient to insert the cells of the battery 10 in the correct manner, replacement of the protection fuse F1 being unnecessary.

The power supply 2 preferably further comprises a residual charge measuring stage 28. The residual charge measuring stage 28 comprises a resistive divider formed of resistors R4 and R11 and connected between node A and the negative terminal of the battery 10 or node D. The output signal of the resistive divider, OUT_MEAS in FIG. 3, is proportional to the voltage of the battery 10 (neglecting the internal resistance of the pMOS Q1 and of the resettable protection fuse F1) and it is lowered to a level suitable to be provided, through a connection 29, to an analog input of the microcontroller 5, which may thus measure with a good precision the voltage of the battery 10 and consequently estimate the residual charge of the battery 10.

The evaluation by the microcontroller 5 of the residual energy in the cells of the battery 10 allows, for example, to decide whether to start a treatment cycle with a reasonable confidence of being able to end it, and/or to emit a warn signal through the display 13 and/or the buzzer 14 if the residual charge is below a predetermined threshold and it is deemed insufficient to perform the treatment cycle.

Those skilled in the art will understand that the block diagram described above applies in the case of a power supply 2 provided with an internal battery power source. The changes to be made in the case of power supply by the mains are within the skills of those skilled in the art in the light of the above description.

Hereinafter there is described, with the aid of **FIG. 4****,** an embodiment of the driver 6 of the electrodes 4 of pair 8; driver 7, if present, is identical with driver 6.

In the embodiment shown, the driver 6 comprises a power supply stage 30 configured to provide two isolated voltages from the non-isolated stable voltage (e.g. +5V) provided by the power supply 2 between node B and node D of FIG. 3.

A first isolated voltage (e.g. +30V) is provided, between a node E and a node F, by a DC/DC step-up converter or boost converter U7 and it is used, advantageously, to power the analog part of the driver 6. The reference voltage, or isolated ground, provided by the power supply stage 30 is labelled as node F in the diagram of FIG. 4.

In the power supply stage 30, a stabilizer regulator or linear converter U8, preferably but not necessarily of the LDO (Low DropOut) type, starting from the aforementioned output voltage of the boost converter U7 or from a second output voltage thereof, generates said second isolated voltage (e.g. + 5V), between a node G and the node F. The second isolated voltage is advantageously used to power the logic part of the driver 6.

In the case of the example voltage values indicated above, the power supply stage 30 comprises a voltage booster and both isolated stable voltages are higher than the voltage of the battery 10, but in other cases either or both isolated stable voltages may be lower than the voltage delivered by the battery 10.

In order to lower the heat dissipation of linear regulator U8, it is not inputted said first isolated voltage (e.g. +30V) at node E, rather a halved voltage (e.g. +15V) provided at another output of the boost converter U7; however it could be inputted the voltage between nodes E and F.

In other embodiments, the power supply stage 30 could comprise two voltage regulators operating in parallel, rather than cascaded.

In the embodiment shown, the driver 6 comprises an amplitude modulating stage 31, preferably and advantageously comprising a first opto-isolator electrically isolating the pair 8 of electrodes 4 from the power supply 2.

The amplitude modulating stage 31 receives from microcontroller 5, on a connection 32, a signal, IN_SET_V in the diagram of FIG. 4.

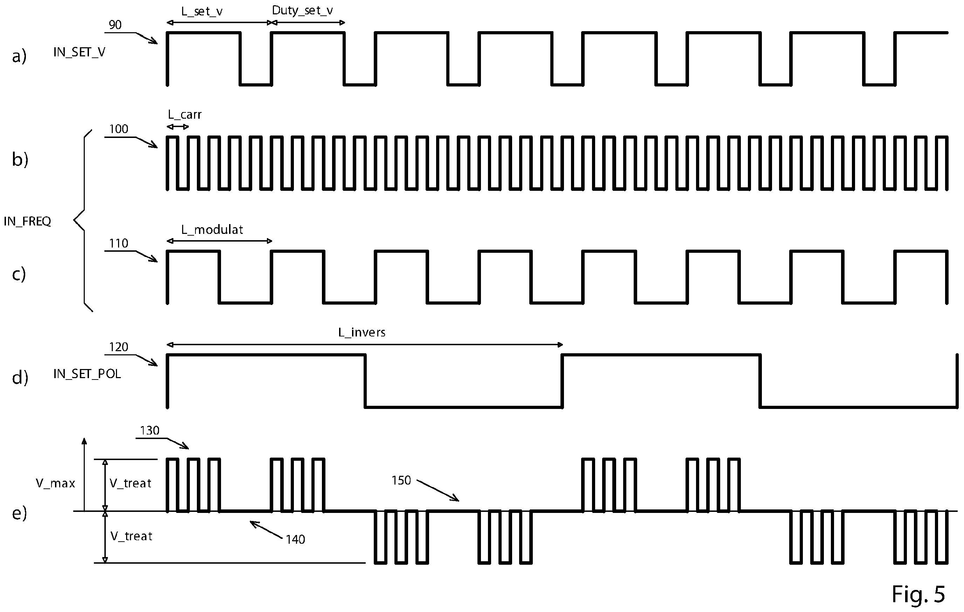

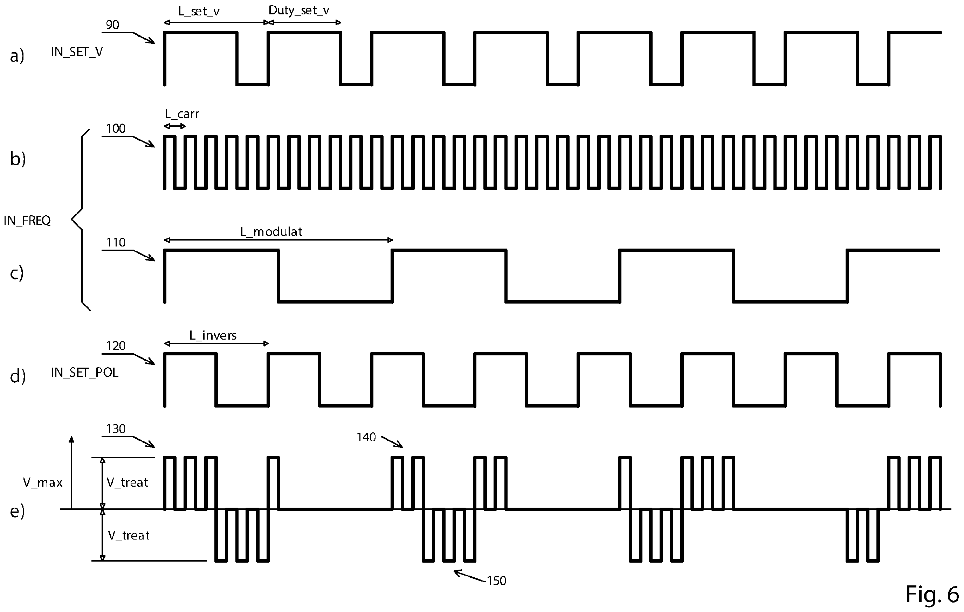

The signal IN_SET_V is preferably a Pulse Width Modulation, PWM, signal, which qualitative trend is shown **FIGs. 5a****,** **6a** better described hereinafter. The microcontroller 5, by changing the duty cycle Duty_set_v of the signal IN_SET_V, may set, through the amplitude modulating stage 31, the amplitude of the voltage V_treat between the pair 8 of electrodes 4 (cf. FIGs. 5e**,** 6e) between a minimum voltage value V_min when the duty cycle Duty_set_v is at 0%, corresponding to the isolated reference voltage at node F, and a maximum voltage value V_max when the duty cycle Duty_set_v is at 100% (cf. FIGs. 5e, 6e), corresponding to the first isolated voltage at node E, for example between 0 and +30V. The frequency F_PWM of the signal PWM is, for example, of 250 Hz. In FIGs. 5a, 6a the period L_set_v of the PWM signal is indicated.

The signal IN_SET_V drives, through a resistor R17, the base of a npn transistor Q9. The transistor Q9 is connected, in series with a resistor R14 and the light-emitting diode of a first photocoupler (or opto-isolator) U3, indicated by reference 33, between node B at the stable voltage provided by power supply 2 and node D at the negative voltage of battery 10. The phototransistor of the first photocoupler U3 is therefore controlled by the signal IN_SET_V, but the components downstream of the opto-isolator U3 33 are electrically isolated from the battery 10, so that the passage of eddy currents is prevented.

The output of the phototransistor of the first photocoupler 33 (U3), namely the signal on its emitter, is buffered by a voltage follower 34 formed by transistors Q5 and Q7 and by resistor R16. The voltage follower 34 is connected between the node at the comparatively high isolated voltage (e.g., +30V) and the node F at the isolated reference voltage (e.g., 0V), so that on the output node of the voltage follower 34 (on the two emitters of the transistors Q5 and Q7) there is a signal with maximum amplitude of 30V, modulated according to the waveform of the signal IN_SET_V present on the connection 32, namely modulated with a PWM signal having the frequency F_PWM (fixed) and the duty cycle Duty_set_V set by the microcontroller 5, in the range between 0 and 100% - or in a more limited range, if desired.

The signal present at the output node of the voltage follower 34 passes through a low pass filter 35 formed by resistor R15 and by capacitor C4. The low pass filter 35 is used to convert the PWM modulation into a direct voltage. In other words, at the output of the low pass filter 35, at the ends of capacitor C4, there is a direct voltage which amplitude is related to the comparatively high isolated voltage (node E, e.g. +30V) by a proportionality factor equal to the duty cycle Duty_set_V, and therefore variable between V_min (e.g. 0V) for a duty cycle of 0%, and a maximum value V_max (e.g. +30V) for a duty cycle of 100%.

The direct voltage signal downstream of the low pass filter 35 is buffered by a voltage follower 36 formed by the transistors Q6 and Q8. The voltage follower 36 is connected between the node E at the comparatively high isolated voltage (e.g., + 30V) and the node F at the isolated reference voltage, so that on the output node of the voltage follower 36 (on the two emitters of transistors Q6 and Q8) and therefore at the output of the amplitude modulating stage 31 there is a direct voltage signal having an amplitude V_treat whose value is variable between V_min and V_max (e.g. between 0V and + 30V), the signal V_treat being used by the subsequent stages of the driver 6 as described below.

In the embodiment shown, driver 6 further comprises a first isolation stage 37.

The isolation stage 37 receives from the microcontroller 5, on a connection 38, a signal, IN_FREQ, which allows the waveform generated at the electrodes 4 by the driver 6 to be controlled.

The signal IN_FREQ is obtained by modulating a carrier square wave, having a preselected frequency (and a duty cycle of 50%), a schematic representation of which is shown in **FIGs. 5b****,** **6b** as a carrier square wave 100, with another square wave having a lower frequency, a schematic representation of which is shown in **FIGs. 5c****,** **6c** as a modulating wave 110.

The carrier square wave 100, having a period L_carr (FIGs 5b, 6b), is obtained by dividing, by a desired factor, the clock frequency of microcontroller 5, generated by an PC oscillator present within microcontroller 5 or preferably by an oscillator internal to microcontroller 5 which uses a quartz crystal (not shown).

The modulating square wave, having a period L_modulat (FIGG. 5c, 6c), is also obtained by dividing, by a desired factor, the clock frequency of microcontroller 5. As better described hereinafter, the microcontroller 5 is configured to change the dividend of the clock frequency during a treatment session, so as to generate modulating square waves having different modulation periods L_modulat during the treatment session.

The signal IN_FREQ (not shown) is represented by the combination of the waves of FIGs. 5b and 5c, respectively of FIGs. 6b, 6c.

Turning back to FIG. 4, the isolation stage 37 of the driver 6 comprises, preferably and advantageously, a second photocoupler 39 (U4) for electrical isolation of the pair 8 of electrodes 4 from the power supply 2. The second photocoupler 39 is analogous to and connected in a similar way to the first photocoupler 33 described above, the description of which is referred to for the sake of brevity. Through the photocoupler 39 (U4), the components downstream of the photocoupler U4 39 are electrically isolated from the battery 10, so that the passage of eddy currents is prevented.

In the embodiment shown, driver 6 further comprises a second isolation stage 40.

The second isolation stage 40 receives from the microcontroller 5, on a connection 41, a signal, IN_SET_POL in the diagram of FIG. 4, which allows the microcontroller 5 to control, over time, the polarity of the voltage V_treat (FIGs. 5e, 6e) generated by the driver 6 at the ends of the electrodes 4 of pair 8.

Signal IN_SET_POL is a square wave at a predetermined frequency (period L_invers), having a duty cycle of 50%, which qualitative progression is shown in **FIGG. 5d, 6d** later described.

The second isolation stage 40 of driver 6 comprises, preferably and advantageously, a third photocoupler 42 (U6) for electrically isolating the pair 8 of electrodes 4 from the power supply 2. The third photocoupler 42 is analogous to and it is connected in an analogous manner to the above described first photocoupler 33, to which description reference is made for the sake of brevity.

In the embodiment shown, the driver 6 further comprises two output stages 43, 44, which are identical to each other.

The first output stage 43 essentially comprises an enabling stage 45 comprising the transistor Q11 and the resistors R18, R20, a voltage follower 46 comprising the transistors Q10 and Q13, and a final stage 47 comprising the transistor Q14, the diode D2 and the resistors R21, R24, R26.

The second output stage 44 essentially comprises an enabling stage 48 comprising the transistor Q16 and the resistors R28, R31, a voltage follower 49 comprising the transistors Q15 and Q18, and a final stage 50 comprising the transistor Q19, the diode D3 and the resistors R32, R35, R36.

The operation of the output stages 43, 44 is explained below.

In the embodiment shown, the driver 6 comprises a logic stage 51 which suitably combines the signals IN_ FREQ and IN_SET_POL provided on the two connections 38, 41 by the microcontroller 5 and electrically isolated by the isolation stages 37, 40. The logic stage 51 comprises, in the embodiment shown, four logic gates NAND U5A, U5B, U5C, U5D suitably connected with each other.

When the signal IN_SET_POL inputted on connection 41 has a first value corresponding to a binary "1", indicative of the fact that the voltage at the electrodes 4 need not be inverted, and therefore the generated waveform must be positive, the logic stage 51 controls, through the NAND gate U5A, the transistor Q11 of the first output stage 43 with the wave form of the signal IN_FREQ inputted on connection 38. Therefore, on the emitters of the voltage follower 46, this waveform will be present, with an amplitude controlled by the duty cycle Duty_set_v of the signal IN_SET_V as mentioned above. The logic stage 51 also deactivates the transistor Q14 through the NAND gate U5C.

The logic stage 51, moreover, through the cascade of NAND gates U5C and U5B, activates the transistor Q16 of the second output stage 44, independently of the signal IN_FREQ provided on connection 38. Therefore, on the emitters of the voltage follower 49 there will be the isolated reference voltage (0V).

The logic stage 51, moreover, also activates transistor Q19, through the cascade of NAND gates NAND U5C and U5D.

Under these conditions, the positive voltage at the output of the voltage follower 46 of the first output stage 43, having a magnitude controlled by the duty cycle Duty_set_v of signal IN_SET_V and a waveform controlled by signal IN_FREQ, sets up at the respective pin of connector 19 for electrodes 4, through the diode D2 of the final stage 47.

The other pin of the connector 19 for the electrodes 4, associated with the second output stage 44, is connected to the isolated reference voltage (0V) through the resistor R35 and the transistor Q19. The diode D3 of the final stage 50 of the second output stage 47 causes that, even if the voltage at the output of the voltage follower 49 is connected to the isolated reference voltage (0V), this pin and therefore the corresponding electrode 4 is not forced to this isolated reference voltage (0V) through the resistor R32, rather remains floating, so that the current can flow from the electrostimulator 1 to the patient, but not vice versa.

The desired waveform, having an amplitude controlled by the signal IN_SET_V and a waveform controlled by the signal IN_FREQ, is therefore actually applied to the pair 8 of electrodes 4 with positive polarity. See, for example, the region 140 of the treatment waveform 130 shown in FIGS. 5e, 6e.

*Mutatis mutandis,* in order to generate a negative voltage at the electrodes 4, the logic stage 51 activates the transistors Q11 and Q14 of the first output stage 43 through the NAND gates U5A and U5C, respectively; in the second output stage 44, the transistor Q16 is controlled, through the NAND gate U5B, with the waveform of the signal IN_FREQ present on connection 38, while the transistor Q19 is deactivated through the cascade of NAND gates U5C and U5D. In this way, the pin of the connector 19 for the electrodes 4 associated with the first output stage 43 is connected to the isolated reference voltage (0V), while on the pin of the connector 19 for the electrodes 4 associated with the second output stage 44 there is the positive signal generated by the voltage follower 49, having an amplitude controlled by the duty cycle Duty_set_v of the signal IN_SET_V and a waveform controlled by the signal IN_FREQ.

The desired waveform, having an amplitude controlled by the signal IN_SET_V and a waveform controlled by the signal IN_FREQ, is therefore actually applied to the pair 8 of electrodes 4 with negative polarity. See, for example, the region 150 of the treatment waveform 130 shown in FIGs. 5e, 6e.

The outputs of the output stages 43, 44 are connected to each other through a resistor R27 having a comparatively very large resistance (compared to the output resistance of the driver 6), of the order of magnitude of hundreds of KOhm, which allows any static energy which could accumulate on the electrodes 4 to be discharged, thus avoiding the patient the possible and annoying sensation of static shock when he /she approaches the electrodes 4 to the body skin.

The first, second and third photocoupler 33, 39, 42 advantageously allow the pair 8 of electrodes 4 and the other components of driver 6 to be electrically isolated from the battery 10. Therefore, the patient's safety against current circulation along undesired paths is ensured. The use of photocouplers allows the generation of the waveforms of treatment voltage V_treat to be optimized, also at a very low modulating frequency F_MOD, because the coupling between the microcontroller 5 and the two treatment channels is made with a pass band which, starting from direct, reaches up to a few tens of KHz. Therefore, advantageously distortions of the generated waveforms are avoided, which could occur, especially at low modulation frequencies, if the coupling were of an inductive type.

However, those skilled in the art will understand that the three photocouplers 33, 39, 42 could be replaced by one or two or even more than three photocouplers, making the necessary changes to the circuit diagram of the driver, in a manner which will be apparent to those skilled in the art in the light of the present description. Moreover, those skilled in the art will understand that instead of distinct photocouplers, a commercial module comprising a plurality of photocouplers may be used.

The use of a power supply stage 30 configured to provide two isolated voltages, as mentioned above, similarly features some of the advantages described above.

**FIG. 5****,** which has already been referred to above, schematically shows, in a), b), c), d), the trend over time of some waveforms involved in the generation of the treatment waveform, shown in e), with a first set of values of respective parameters. It shall be understood that the figure is greatly off scale.

The waveform 90 shown at letter a) is representative of the amplitude modulation signal IN_SET_V, having period L_set_v and duty cycle Duty_set_v settable by the microcontroller 5 and provided to driver 6 on connection 32.

The waveform shown at letter b) is representative of a pulsed voltage, carrier square wave, having a comparatively high pulse frequency, for example of about 8-12 kHz. As said, such carrier square wave 100 is generated by the internal clock of microcontroller 5 through use of a suitable oscillator, preferably a quartz one. The pulses of the carrier square wave 100 are unipolar (in particular positive) and the carrier square wave 100 has a predetermined duty cycle. In other words, the voltage is at a positive value for a first part of each period of the carrier L_carr, and is at null value for a second part. Preferably the duty cycle of the carrier square wave 100 is of 50%, so that said first and second parts have a same duration over time.

The carrier square wave 100 is subjected to on and off amplitude modulation, with a comparatively low frequency, as schematically shown by the waveform shown at letter c), representative of a modulating wave 110. Therefore the pulse train of the carrier wave 100 is on for a first part of each modulation period L_modulat and it is off for a second part of the modulation period L_modulat. Preferably the modulating wave 110 has a predetermined duty cycle, preferably of 50%.

As already discussed, the square waves 100 and 110 are not materially present, in that they are "generated" and combined internally of microcontroller 5. The signal IN_FREQ on connection 38 corresponds to such a combination of the waveforms 100 and 110.

The carrier square wave 100 is also subjected to polarity inversion, by reversing the polarity of the signal at the electrodes 4, as schematically shown by a waveform shown at letter d), representative of an inverting signal 120. The inversion period L_invers is for example of about 1 second (namely, an inversion frequency of 1 Hz). The inverting signal 120 corresponds to signal IN_SET_POL on connection 41 and to the waveforms derived therefrom by the second isolation stage 40.

The waveform representative of the treatment signal 130 is shown at letter e). The treatment signal 130 corresponds to the signal supplied, between the pins of connector 19, by each of the output stages 43, 44.

**FIG. 5** represents the case in which the modulation period L_modulat is smaller than the inversion period L_invers or, in other words, in which the modulating frequency F_MOD is higher than the inversion frequency.

The treatment signal 130 comprises a sequence of bursts of pulses of a first polarity, alternating with a sequence of bursts of pulses of opposite polarity, the successive bursts of pulses in each sequence being separated by a respective period of absence of stimulation; and all the above repeats itself over time.

**FIG. 6** represents the same waveforms of FIG. 5, however in the case in which the modulation period L_modulat is greater than the inversion period L_invers, or in other words, in which the modulating frequency is lower than the inversion frequency.

The treatment signal 130 comprises a sequence of immediately successive bursts of pulses of alternating polarity, followed by a period of absence of stimulation; and all the above repeats itself over time.

As highlighted above, the use of photocouplers, such as photocouplers 33, 39, 42, allows the generation of the waveform 130 of treatment voltage V_treat to be optimized, even at a very low modulating frequency F_MOD, as schematized in FIG. 6, because the coupling between the microcontroller 5 and the two treatment channels is made with a pass band which, starting from direct, reaches up to a few tens of KHz, what advantageously avoids distortions of the waveform 130.

Each treatment session has preferably a predetermined duration, for example settable between a minimum and a maximum duration, for example between 4 seconds and 4 hours, for example about twenty minutes.

Preferably a treatment session comprises a plurality of sub-phases, for example up to four sub-phases, wherein the same carrier frequency is preferably (but not necessarily) used, for example at 10KHz, and each of which has a duration which preferably can be set between a minimum duration and a maximum duration, for example between 1 and 3600 seconds.

In each sub-phase, the modulating frequency F_MOD is varied, namely the period L_modulat of the modulating signal 110.

The sub-phases can have the same time durations or different time durations.

The sub-phases can be immediately subsequent to each other or between at least two of the sub-phases a rest time interval can be provided, wherein the carrier signal 100 (or the related derived form IN_FREQ) is off.

Preferably, in the first three sub-phases of the treatment cycle a modulating frequency F_MOD higher than the inversion frequency is used, obtaining a waveform of the treatment signal as shown in FIG. 5e), while in the last sub-phase of the treatment cycle a modulating frequency F_MOD lower than the inversion frequency is used, obtaining a waveform of the treatment signal as shown in FIG. 6e).

Merely by way of an example, a treatment cycle can comprise four sub-phases, in which a carrier frequency at 10KHz is used and each of which has the duration and modulating frequency indicated below:
- modulating frequency 290-330 Hz, for example 330 Hz, for a duration of about 1 minute
- modulating frequency 25-35 Hz, for example 31 Hz, for a duration of about 2 minutes
- modulating frequency 7-10 Hz, for example 9 Hz, for a duration of about 7 minutes
- modulating frequency 0,1-1, for example 0,28 Hz, for a duration of about 10 minutes.

With the exemplary numerical data indicated above, the delivered pulsed direct current has a peak value ranging for example from 1 to 800 microamps, assuming a resistance of the body of the treated subject, between the electrodes 4, of about 500 ohms.

Again merely as an example, in each sub-phase the inversion period L_invers may be comprised between 430 and 550 millisec, namely the inversion frequency is comprised between 0.9 and 1.16 Hz, for example it is 1, 04 Hz.

The minimum, average and/or maximum values of voltage and/or current, the modulation frequencies, the durations of the individual sub-phases, etc. may be stored in memory 15 and/or communicated outside the electrostimulator 1 through the communication interface 17.

The microcontroller 5 of the electrostimulator 1 may be configured to have specific or additional functions (some of which have been mentioned above also), among which:
- display and user interface
- Bluetooth communication interface (whit medical staff and/or with the patient)
- customized communication protocol
- remote control, through app on a tablet, smartphone, PC
- parametrizable in the duration of each sequence
- parametrizable in the frequencies, duty cycle, modulations and polarity inversion times
- possibility of regulating the voltage intensity delivered on each channel, in an analog manner or by levels (for example, 50 levels per channel)
- possibility of pausing or stopping and restoring a treatment cycle
- auto-off when one or more conditions occur
- possibility of storing a plurality of users
- differentiated privileges based on the type of user (medical staff and assistance staff, possibly also patients)
- possibility of storing the treatment log, with one or more of the following data: date, time (thanks to the RTC 16), number of completed sequencies, average value and maximum value of the voltage intensity for each channel and each sequence
- possibility of downloading the logs onto a processor through a serial interface
- possibility of directly communicating with a networked server, Internet included
- various sound indications
- control of the residual charge of the batteries and inhibiting to start a session if it is insufficient (through the residual charge measuring stage 28) The various alternative embodiments, variants and/or possibilities of each component or group of components that have been described are to be meant as combinable with each other in any manner, unless they are mutually incompatible.

The above is a description of various embodiments, variants and/or possibilities of inventive aspects, and further changes can be made without departing from the scope of the present invention. The shape and/or size and/or location and/or orientation of the various components and/or the succession of the various steps can be changed. The functions of an element or module can be carried out by two or more components or modules, and vice-versa. Components shown directly connected to or contacting each other can have intermediate structures arranged in between them. Steps shown directly following each other can have intermediate steps carried out between them. The details shown in a figure and/or described with reference to a figure or to an embodiment can apply in other figures or embodiments. Not all of the details shown in a figure or described in a same context must necessarily be present in a same embodiment. Features or aspects that turn out to be innovative with respect to the prior art, alone or in combination with other features, should be deemed to be described *per se,* irrespective of what is explicitly described as innovative.

## Claims

1. Electrostimulator for vision (1), comprising:
- at least one pair (8, 9) of electrodes (4) configured to make contact with the skin of a subject to be treated,
- a voltage power supply (2) configured to provide at least one stable voltage,
- a waveform generator (3) configured to generate, from said at least one stable voltage, at least one voltage signal to be applied to said electrodes (4), said at least one voltage signal having a preselected waveform (130), so as to deliver a treatment current to the subject,
wherein the waveform generator (3) comprises microcontroller (5) and at least one driver (6, 7) of said at least one pair (8, 9) of electrodes (4), **characterized in that** the waveform generator (3) comprises at least one photocoupler (33, 39, 42) for electrically isolating said at least one pair (8, 9) of electrodes (4) from said voltage power supply (2), and **in that** said at least one driver (6, 7) comprises said at least one photocoupler (33, 39, 42) at the interface with the microcontroller (5).

2. Electrostimulator for vision (1) according to claim 1, wherein said at least one driver (6, 7) comprises an amplitude modulation stage (31) comprising a photocoupler (33) of said at least one photocoupler (33, 39, 42).

3. Electrostimulator for vision (1) according to claim 2, wherein said amplitude modulation stage (31) is driven by a signal (90, IN_SET_V) emitted by said microcontroller (5), preferably by a Pulse Width Modulation, PWM, signal.

4. Electrostimulator for vision (1) according to any of claims 1-3, wherein said at least one driver (6, 7) comprises at least one and preferably two isolation stages (37, 40), each one comprising a respective photocoupler (39, 42) of said at least one photocoupler (33, 39, 42).

5. Electrostimulator for vision (1) according to claim 4, wherein one of said isolation stages (37) is driven by said microcontroller (5) with a waveform (IN_FREQ) obtained by modulating a carrier square wave (100) with a modulating square wave (110) having a modulating frequency (F_MOD) lower than the frequency of said carrier square wave (100).

6. Electrostimulator for vision (1) according to any of claims 4-5, wherein one of said isolation stages (40) is driven by said microcontroller (5) with a square waveform (120, IN_SET_POL) for reversing the polarity of said voltage signal having a preselected waveform (130).

7. Electrostimulator for vision (1) according to any of claims 1-6, wherein said at least one driver (6, 7) comprises a power supply stage (30) configured to provide at least one stable voltage electrically isolated from a voltage supplied by said voltage power supply (2).

8. Electrostimulator for vision (1) according to any of claims 1-7, wherein said voltage power supply (2) comprises a power source (10) and at least one stabilizer regulator (U1) to provide at least one stable voltage.

9. Electrostimulator for vision (1) according to any of claims 1-8, wherein said voltage power supply (2) comprises a switching on/off stage (24) driven by said microcontroller (5).

10. Electrostimulator for vision (1) according to claim 9, wherein said voltage power supply (2) comprises a protection stage (27) of said switching on/off stage (24) against overload and polarity inversion of one or respectively of said power source (10) of the voltage power supply (2).

11. Electrostimulator for vision (1) according to any of claims 1-10, wherein said voltage power supply (2) comprises one or respectively said power source (10) and a residual charge measuring stage (28) of said power source (10).

12. Electrostimulator for vision (1) according to claim 5 or any of claims 6-11 when depending on claim 5, wherein said microcontroller (5) is configured to use a different modulating frequency (F_MOD) in each of a plurality of sub-phases of a treatment cycle.

## Patentansprüche

1. Elektrostimulator für die Sehkraft (1), umfassend:
- mindestens ein Paar (8, 9) von Elektroden (4), die konfiguriert sind, um mit der Haut eines zu behandelnden Subjekts in Kontakt zu treten,
- eine Spannungsstromversorgung (2), die konfiguriert ist, um mindestens eine stabile Spannung bereitzustellen,
- einen Wellenformgenerator (3), der dazu konfiguriert ist, aus der mindestens einen stabilen Spannung mindestens ein an die Elektroden (4) anzulegendes Spannungssignal zu erzeugen, wobei das mindestens eine Spannungssignal eine vorgewählte Wellenform (130) aufweist, um dem Subjekt einen Behandlungsstrom zuzuführen,
wobei der Wellenformgenerator (3) einen Mikrocontroller (5) und mindestens einen Treiber (6, 7) des mindestens einen Paars (8, 9) von Elektroden (4) umfasst, **dadurch gekennzeichnet, dass** der Wellenformgenerator (3) mindestens einen Photokoppler (33, 39, 42) umfasst, um das mindestens eine Paar (8, 9) von Elektroden (4) elektrisch von der Spannungsstromversorgung (2) zu isolieren, und dadurch, dass der mindestens eine Treiber (6, 7) den mindestens einen Photokoppler (33, 39, 42) an der Schnittstelle zum Mikrocontroller (5) umfasst.

2. Elektrostimulator für die Sehkraft (1) nach Anspruch 1, wobei der mindestens eine Treiber (6, 7) eine Amplitudenmodulationsstufe (31) umfasst, die einen Photokoppler (33) des mindestens einen Photokopplers (33, 39, 42) umfasst.

3. Elektrostimulator für die Sehkraft (1) nach Anspruch 2, wobei die Amplitudenmodulationsstufe (31) durch ein Signal (90, IN_SET_V) angetrieben wird, das von dem Mikrocontroller (5) emittiert wird, vorzugsweise durch ein Pulsweitenmodulations-, PWM-, Signal.

4. Elektrostimulator für die Sehkraft (1) nach einem der Ansprüche 1-3, wobei der mindestens eine Treiber (6, 7) mindestens eine und vorzugsweise zwei Isolationsstufen (37, 40) umfasst, wobei jede einen jeweiligen Photokoppler (39, 42) des mindestens einen Photokopplers (33, 39, 42) umfasst.

5. Elektrostimulator für die Sehkraft (1) nach Anspruch 4, wobei eine der Isolationsstufen (37) durch den Mikrocontroller (5) mit einer Wellenform (IN_FREQ) angetrieben wird, die durch Modulieren einer Trägerrechteckwelle (100) mit einer modulierenden Rechteckwelle (110) mit einer Modulationsfrequenz (F_MOD), die niedriger als die Frequenz der Trägerrechteckwelle (100) ist, erhalten wird.

6. Elektrostimulator für die Sehkraft (1) nach einem der Ansprüche 4-5, wobei eine der Isolationsstufen (40) durch den Mikrocontroller (5) mit einer Rechteckwellenform (120, IN_SET_POL) zum Umkehren der Polarität des Spannungssignals mit einer vorgewählten Wellenform (130) angetrieben wird.

7. Elektrostimulator für die Sehkraft (1) nach einem der Ansprüche 1-6, wobei der mindestens eine Treiber (6, 7) eine Stromversorgungsstufe (30) umfasst, die konfiguriert ist, um mindestens eine stabile Spannung bereitzustellen, die elektrisch von einer Spannung isoliert ist, die von der Spannungsstromversorgung (2) geliefert wird.

8. Elektrostimulator für die Sehkraft (1) nach einem der Ansprüche 1-7, wobei die Spannungsstromversorgung (2) eine Stromquelle (10) und mindestens einen Stabilisatorregler (U1) umfasst, um mindestens eine stabile Spannung bereitzustellen.

9. Elektrostimulator für die Sehkraft (1) nach einem der Ansprüche 1-8, wobei die Spannungsstromversorgung (2) eine Ein-/Ausschaltstufe (24) umfasst, die von dem Mikrocontroller (5) angetrieben wird.

10. Elektrostimulator für die Sehkraft (1) nach Anspruch 9, wobei die Spannungsstromversorgung (2) eine Schutzstufe (27) der Ein-/Ausschaltstufe (24) gegen Überlastung und Polaritätsumkehr einer oder jeweils der Stromquelle (10) der Spannungsstromversorgung (2) umfasst.

11. Elektrostimulator für die Sehkraft (1) nach einem der Ansprüche 1-10, wobei die Spannungsstromversorgung (2) eine oder jeweils die Stromquelle (10) und eine Restladungsmessstufe (28) der Stromquelle (10) umfasst.

12. Elektrostimulator für die Sehkraft (1) nach Anspruch 5 oder einem beliebigen der Ansprüche 6-11, wenn abhängig von Anspruch 5, wobei der Mikrocontroller (5) dazu konfiguriert ist, eine unterschiedliche Modulationsfrequenz (F_MOD) in jeder einer Vielzahl von Unterphasen eines Behandlungszyklus zu verwenden.

## Revendications

1. Électrostimulateur pour la vision (1), comprenant:
- au moins une paire (8, 9) d'électrodes (4) configurées pour entrer en contact avec la peau d'un sujet à traiter,
- une alimentation en tension (2) configurée pour fournir au moins une tension stable,
- un générateur de forme d'onde (3) configuré pour générer, à partir de ladite au moins une tension stable, au moins un signal de tension à appliquer auxdites électrodes (4), ledit au moins un signal de tension ayant une forme d'onde présélectionnée (130), de manière à délivrer un courant de traitement au sujet,
dans lequel le générateur de forme d'onde (3) comprend un microcontrôleur (5) et au moins un mandrin (6, 7) de ladite au moins une paire (8, 9) d'électrodes (4), **caractérisé en ce que** le générateur de forme d'onde (3) comprend au moins un photocoupleur (33, 39, 42) pour isoler électriquement ladite au moins une paire (8, 9) d'électrodes (4) de ladite alimentation en tension (2), et **en ce que** ledit au moins un mandrin (6, 7) comprend ledit au moins un photocoupleur (33, 39, 42) à l'interface avec le microcontrôleur (5).

2. Électrostimulateur pour la vision (1) selon la revendication 1, dans lequel ledit au moins un mandrin (6, 7) comprend un étage de modulation d'amplitude (31) comprenant un photocoupleur (33) dudit au moins un photocoupleur (33, 39, 42).

3. Électrostimulateur pour la vision (1) selon la revendication 2, dans lequel ledit étage de modulation d'amplitude (31) est entraîné par un signal (90, IN_SET_V) émis par ledit microcontrôleur (5), de préférence par un signal de modulation de largeur d'impulsion (PWM).

4. Électrostimulateur pour la vision (1) selon l'une quelconque des revendications 1 à 3, dans lequel ledit au moins un mandrin (6, 7) comprend au moins un et de préférence deux étages d'isolation (37, 40), chacun comprenant un photocoupleur (39, 42) respectif dudit au moins un photocoupleur (33, 39, 42).

5. Électrostimulateur pour la vision (1) selon la revendication 4, dans lequel l'un desdits étages d'isolation (37) est entraîné par ledit microcontrôleur (5) avec une forme d'onde (IN_FREQ) obtenue en modulant une onde carrée porteuse (100) avec une onde carrée modulante (110) ayant une fréquence de modulation (F_MOD) inférieure à la fréquence de ladite onde carrée porteuse (100).

6. Électrostimulateur pour la vision (1) selon l'une quelconque des revendications 4 à 5, dans lequel l'un desdits étages d'isolation (40) est entraîné par ledit microcontrôleur (5) avec une forme d'onde carrée (120, IN_SET_POL) pour inverser la polarité dudit signal de tension ayant une forme d'onde présélectionnée (130).

7. Électrostimulateur pour la vision (1) selon l'une quelconque des revendications 1 à 6, dans lequel ledit au moins un mandrin (6, 7) comprend un étage d'alimentation (30) configuré pour fournir au moins une tension stable électriquement isolée d'une tension fournie par ladite alimentation en tension (2).

8. Électrostimulateur pour la vision (1) selon l'une quelconque des revendications 1 à 7, dans lequel ladite alimentation en tension (2) comprend une source d'alimentation (10) et au moins un régulateur stabilisateur (U1) pour fournir au moins une tension stable.

9. Électrostimulateur pour la vision (1) selon l'une quelconque des revendications 1 à 8, dans lequel ladite alimentation en tension (2) comprend un étage de commutation marche/arrêt (24) entraîné par ledit microcontrôleur (5).

10. Électrostimulateur pour la vision (1) selon la revendication 9, dans lequel ladite alimentation en tension (2) comprend un étage de protection (27) dudit étage de commutation marche/arrêt (24) contre la surcharge et l'inversion de polarité d'une ou respectivement de ladite source d'alimentation (10) de l'alimentation en tension (2).

11. Électrostimulateur pour la vision (1) selon l'une quelconque des revendications 1 à 10, dans lequel ladite alimentation en tension (2) comprend une ou respectivement ladite source d'alimentation (10) et un étage de mesure de la charge résiduelle (28) de ladite source d'alimentation (10).

12. Électrostimulateur pour la vision (1) selon la revendication 5 ou l'une quelconque des revendications 6 à 11 lorsqu'elles dépendent de la revendication 5, dans lequel ledit microcontrôleur (5) est configuré pour utiliser une fréquence de modulation différente (F_MOD) dans chacune d'une pluralité de sous-phases d'un cycle de traitement.
